# EUROPEAN PATENT SPECIFICATION

(11) **EP 4 039 227 B1**
(45) Date of publication and mention of the grant of the patent: **15.04.2026**
(21) Application number: 20871442.8
(22) Date of filing: 28.09.2020
(51) Int. Cl.: A61F 2/12, A61F 2/00, A61L 27/50, A61L 27/14, B33Y 80/00

(54) **PATIENT-SPECIFIC BREAST IMPLANT FOR BREAST RECONSTRUCTION AFTER BREAST-CONSERVING MASTECTOMY**
PATIENTENSPEZIFISCHES BRUSTIMPLANTAT ZUR BRUSTREKONSTRUKTION NACH BRUSTERHALTENDER MASTEKTOMIE
IMPLANT MAMMAIRE SPÉCIFIQUE AU PATIENT POUR LA RECONSTRUCTION MAMMAIRE APRÈS MASTECTOMIE CONSERVATRICE DU SEIN

(30) Priority: 02.10.2019 KR 20190122341
(43) Date of publication of application: 10.08.2022
(73) Proprietor: Konyang University Industrial Cooperation Group, Chungcheongnam-do 32992 (KR); Osong Medical Innovation Foundation, Cheongju-si, Chungcheongbuk-do 28160 (KR)
(72) Inventor: KIM, Hoon, Seoul 06598 (KR); PARK, Ji Sun, Sejong 30062 (KR); JUNG, Tae Gon, Cheongju-si Chungcheongbuk-do 28162 (KR); LEE, Su Jeong, Daejeon 35211 (KR)
(74) Representative: Witte, Weller & Partner Patentanwälte mbB
(86) International application number: PCT/KR2020/013252
(87) International publication number: WO 2021/066472

(56) References cited:
- KR-A- 20130 004 575
- KR-A- 20190 046 465
- KR-A- 20190 062 456
- KR-A- 20190 101 173
- KR-B1- 101 051 014

## Description

### Technical Field

The present invention relates to a patient-specific breast implant and. More particularly, the present invention relates to a patient-specific breast implant which, when a breast-conserving surgery (BCS, also called lumpectomy or partial mastectomy) is performed to remove breast cancer while leaving as much normal breast tissue as possible, prevents deformation of breast due to the depression of breast skin or adhesions of remaining breast tissues after the BCS, shortens surgery time, and facilitates securing breast symmetry, thereby ultimately dramatically increasing the success rate of surgery and maximizing patient satisfaction.

### Background Art

Modified Radical Mastectomy (MRM) is an operation to remove the entire breast for the treatment of breast cancer. After the modified radical mastectomy, breast reconstruction is performed to reconstruct the removed breast.

A tissue expander is a device used in the breast reconstruction surgery. The tissue expander is first placed under the skin and then gradually expanded to secure a space under the skin. That is, such a device has been used to form a breast pocket for accommodating a permanent breast implant or used for skin grafting or reconstruction.

The tissue expanders for breast reconstruction surgery have been used to create a breast pocket to accommodate a permanent breast implant. These expanders are typically in the form of a silicone shell. After the breast pocket is created, a fluid, such as saline, is periodically injected into the silicone shell through an injection port until the breast pocket increases to reach a desired size.

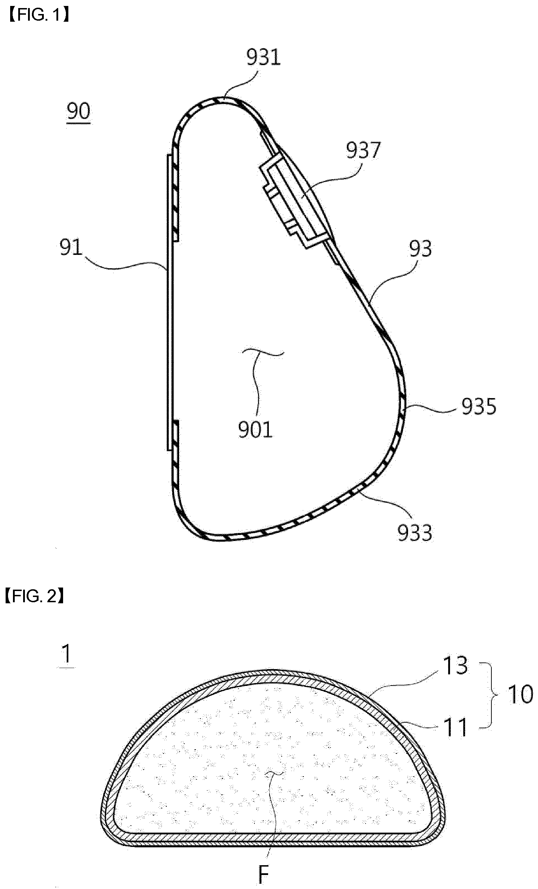

FIG. 1 is a view illustrating a conventional breast tissue expander 90, which is disclosed in Korean Patent Application Publication No. 10-2016-0142349 (December 12, 2016);

Referring to FIG. 1, a conventional breast tissue expander 90 has a posterior surface 91 substantially flat and disposed against the chest wall of the patient, and an anterior surface 93 formed to be convex toward the front side from the chest wall.

The anterior surface 93 is generally made of a silicone material. The anterior surface 93 includes: an upper pole region 931 that is the upper portion of the shell when the breast implant recipient is in a standing posture; a lower pole region 933 that is the lower portion of the shell when the breast implant recipient is in a standing posture; a vertex 935 bordering the upper pole region 931 and the lower pole region 933; and an injection port 937 through which a fluid such as saline is injected into the inner space 901 of the breast tissue expander 90 at regular time intervals.

The conventional breast tissue expander 90 having the configuration described above is inserted into the breast area where total mastectomy has been performed, and then expanded by saline injected at regular intervals so that the skin of the removed breast area can be expanded.

After the cancer treatment is finished, the breast tissue expander 90 inflated like a water balloon is removed, and a breast implant having a shape similar to that of the opposite breast is inserted to rebuild the resected breast.

However, in recent years, as life expectancy after breast cancer treatment has increased, the cosmetic aspect of the breast has been emphasized. For this reason, breast-conserving surgery (lumpectomy or partial mastectomy) which removes only breast cancer tissue and minimizes the removal of surrounding healthy tissue is widely used.

Since the breast-conserving surgery (lumpectomy or partial mastectomy) is an operation that partially removes the patient's breast, there is an advantage that the nipple and areola of the patient can be preserved. However, this operation has a problem in that the contour of the entire breast is distorted and the shape of the breast is not aesthetically pleasing.

To solve this problem, various surgical methods such as fat grafting surgery and local flap surgery have been tried, but there is no satisfactory method so far. In particular, the method using the existing breast tissue expander 90 causes the problem of expanding the remaining surrounding breast tissue, resulting in expansion of unwanted areas.

Accordingly, in the related industry, there is a demand for a new technology that allows the shape of the breast after surgery to be similarly reconstructed to the original shape while minimizing the resection of the breast.

(Patent Document 1) Korean Patent Application Publication No. 10-2016-0142349 (as of December 12, 2016) discloses a Directional Tissue Expander, (Patent Document 2) Korean Patent Application Publication No. 2013-0004575 discloses an Inflatable Prostheses and Methods of Making Same, and (Patent Document 3) Korean Patent Application Publication No. 2019-0046465 discloses a Method of Manufacturing Customized Breast Prosthesis Manufacturing System Performing the Same Computer Program and Computer-Readable Record Medium for the Same Customized Breast Prosthesis and Customized Correction Brassiere.

### Disclosure

### Technical Problem

The present invention has been devised to solve the aforementioned problems.

An objective of the present invention is to prevent a breast from being deformed due to depression of the breast skin, adhesions of the remaining breast tissue, etc. after breast-conserving surgery (lumpectomy or partial mastectomy) that is performed to preserve breast tissue as much as possible and to minimize the resection of healthy tissue when removing cancerous tissue.

Another objective of the present invention is to insert a patient-specific breast implant having a shape complementary to the shape of an inner space secured by inserting a small breast tissue expander into a region where breast deformation such as depression and skin contracture has occurred after breast-conserving surgery (lumpectomy or partial mastectomy), thereby shortening the time of breast reconstruction surgery.

A further objective of the present invention is to construct a patient-specific breast implant having a shape complementary to the shape of the inner space secured by inserting a breast tissue expander, thereby remarkably increasing the surgical success rate of breast reconstruction surgery.

A yet further objective of the present invention is to avoid sacrificing additional healthy tissue by inserting a patient-specific breast implant having a shape identical to the shape of an inner space secured by inserting a breast tissue expander.

A yet further objective of the present invention is to construct a shell portion made of a shape memory polymer. In this case, when a patient-specific breast implant having a shape complementary to the shape of a breast tissue removal site after lumpectomy or partial mastectomy is inserted into the body, the shape can be permanently maintained in the body temperature range. This facilitates breast symmetry and enables cosmetically satisfactory breast reconstruction.

A yet further objective of the present invention is to maximize patient satisfaction after surgery by enabling cosmetically satisfactory breast reconstruction even when deformation such as depression and skin contracture occurs after breast-conserving surgery (lumpectomy or partial mastectomy).

A yet further objective of the present invention is to provide a patient-specific breast implant containing a shape-memory polymer that may change in shape with temperature change. When the patient-specific breast implant is implanted in the body, the shape of the breast implant can be permanently maintained in the body temperature range.

A yet further objective of the present invention is to provide a patient-specific breast implant in which a shell portion is made of a shape memory polymer and formed through 3D printing. Thus, when the patient-specific breast implant is implanted into a breast area of a patient, the breast implant changes into a memorized shape so that the breast implant can be seated fitting a space secured by a breast tissue expander.

A yet further objective of the present invention is to provide a breast implant in which a shell portion includes a silicone film made of silicone and a shape memory film made of a shape memory polymer. Since it is not necessary for the entire area of the shell portion is made of a shape memory polymer, the usage of a shape memory polymer that is a relatively expensive material is reduced, resulting in cost reduction of the breast implant.

A yet further objective of the present invention is to provide a breast implant in which a shape memory film is formed on the outer surface of a silicone film through surface treatment so that the overall shape of the breast implant is complementary to the shape of an inner space formed by a breast tissue expander due to the deformation of the shape memory film according to the temperature.

A yet further objective of the present invention is to form a silicone film by 3D printing, so that a silicone film having a shape complementary to the shape of a breast removed during a breast-conserving surgery (i.e., partial mastectomy) process can be formed.

A yet further objective of the present invention is to construct a shell portion and a filler that are made of only a shape memory polymer, so that it is easy to make the breast symmetry even when a narrow area of the breast is depressed and skin contracture occurs after breast-conserving surgery (i.e. partial mastectomy), and it is possible to perform cosmetically satisfactory breast reconstruction.

A yet further objective of the present invention is to form a fixing portion protruding on a shell portion so that a breast implant inserted into an inner space secured by a breast tissue expander can maintain an initial posture as it is installed without being rotated or unintentionally moved in the inner space of the breast inner.

### Technical Solution

In order to achieve the aforementioned objectives, the present invention is implemented by embodiments having configurations in accordance with the presented claims.

The present invention includes a shell portion constituting an outer wall, in which the shell portion has a shape complementary to the shape of an inner space secured by a breast tissue expander.

According to the present invention, the shell portion comprises a shape memory polymer that changes in shape according to temperature change.

According a further embodiment of the present invention, the polymer material may be any one polymer that is certified to be harmless when inserted into the human body and is selected among polynorbornene, polyisoprene, styrene-butadiene copolymer, polyurethane, polyethylene, and polyester-based polymers.

According to a further embodiment of the present invention, the polymer material may be a polyester-based polymer, and the polyester-based polymer may be any one or a combination of two or more that are selected from polyphosphazene, polyanhydride, poly acetal, polyorthoester, polyphosphoester, polyglycolide, poly-ε-caprolactone, polylactide, polycarbonate, and polyamide and which are certified to be harmless when inserted into the human body.

According to a further embodiment of the present invention, the shell portion may include a silicone film formed of silicone and a shape memory film formed of a shape memory polymer.

According to a further embodiment of the present invention, the shape memory film may be formed on the outer surface of the silicone film through surface treatment of the silicone film.

According to a further embodiment of the present invention, the silicone film may be formed through 3D printing.

According to a further embodiment of the present invention, the shell portion may be formed of only the shape memory polymer through 3D printing.

According to a further embodiment of the present invention, both of filler and the shell portion may be made of only the shape memory polymer.

According to a further embodiment of the present invention, both of filler and the shell portion may be formed of only the shape memory polymer through 3D printing.

According to a further embodiment of the present invention, the patient-specific implant may further include a fixing portion protruding from the surface of the shell portion.

### Advantageous Effects

The present invention can obtain effects described below due to the configuration, combination, and use relationship described below and described above with reference to the embodiments.

The present invention has an effect of preventing a breast from being deformed due to depression of the breast skin, adhesions of the remaining breast tissue, etc. after breast-conserving surgery (lumpectomy or partial mastectomy) that is performed to preserve breast tissue as much as possible and to minimize the resection of cancerous tissue.

The present invention enables insertion of a patient-specific breast implant having a shape complementary to the shape of an inner space secured by inserting a small breast tissue expander into a region where breast deformation such as depression and skin contracture has occurred after breast-conserving surgery (lumpectomy or partial mastectomy), thereby providing an effect of shortening the time of breast reconstruction surgery by .

The present invention constructs a patient-specific breast implant having a shape complementary to the shape of the inner space secured by inserting a breast tissue expander, thereby having an effect of remarkably increasing the surgical success rate of breast reconstruction surgery.

The present invention enables insertion of a patient-specific breast implant having a shape identical to the shape of an inner space secured by a breast tissue expander, thereby preventing additional healthy tissue from being sacrificed.

The present invention allows construction of a shell portion made of a shape memory polymer. Thus, when a patient-specific breast implant having a shape complementary to the shape of a breast tissue removal site after lumpectomy or partial mastectomy is inserted into the body, the shape of the implant can be permanently maintained in the body temperature range. This facilitates breast symmetry and enables cosmetically satisfactory breast reconstruction.

The present invention has an effect of maximizing patient satisfaction after surgery by enabling cosmetically satisfactory breast reconstruction even when deformation such as depression and skin contracture occurs after breast-conserving surgery (lumpectomy or partial mastectomy).

The present invention provides a patient-specific breast implant containing a shape-memory polymer that may change in shape with temperature change. When the patient-specific breast implant is implanted in the body, the shape of the breast implant can be permanently maintained when the body temperature range.

The present invention provides a patient-specific breast implant in which a shell portion is made of a shape memory polymer and formed through 3D printing. Thus, when the patient-specific breast implant is implanted into a breast area of a patient, the breast implant changes into a memorized shape so that the breast implant can be seated fitting a space secured by a breast tissue expander.

The present invention provides a breast implant in which a shell portion includes a silicone film made of silicone and a shape memory film made of a shape memory polymer. Since it is not necessary for the entire area of the shell portion is made of the shape memory polymer, the usage of the shape memory polymer that is a relatively expensive material is reduced, resulting in cost reduction of the breast implant.

The present invention provides a breast implant in which a shape memory film is formed on the outer surface of a silicone film through surface treatment so that the overall shape of the breast implant is complementary to the shape of an inner space formed by a breast tissue expander due to the deformation of the shape memory film according to the temperature.

The present invention forms a silicone film through 3D printing, so that a silicone film having a shape complementary to the shape of a breast removed during a breast-conserving surgery (i.e., partial mastectomy) process can be formed.

The present invention constructs a shell portion and a filler that are made of only a shape memory polymer, so that it is easy to make the breast symmetry even when a narrow area of the breast is depressed and skin contracture occurs after breast-conserving surgery (i.e. partial mastectomy), and it is possible to perform cosmetically satisfactory breast reconstruction.

The present invention forms a fixing portion protruding on a shell portion so that a breast implant inserted into an inner space secured by a breast tissue expander can maintain an initial posture as it is installed without being rotated or unintentionally moved in the inner space of the breast inner.

### Description of Drawings

FIG. 1 is a view illustrating a breast tissue expander according to a conventional art;
FIG. 2 is a view illustrating a patient-specific breast implant according to one embodiment of the present invention;
FIG. 3 is a view illustrating an original shape, a temporary shape, and a permanent shape of a shape memory polymer;
FIG. 4 is a view illustrating a patient-specific breast implant having a shell portion made of only a shape memory film, according to another embodiment of the present invention;
FIG. 5 is a view illustrating a patient-specific breast implant made of only a shape memory polymer without using a silicone film or filler, according to a further embodiment of the present invention;
FIG. 6 is a view illustrating a patient-specific breast implant according to a further embodiment of the present invention;
FIG. 7 is a view illustrating a patient-specific breast implant according to a further embodiment of the present invention;
FIG. 8 is a view illustrating a patient-specific breast implant according to a further embodiment of the present invention; and
FIG. 9 is a view illustrating a state in which the present invention is used.

### Best Mode

Hereinafter, patient-specific breast implants according to preferred embodiments of the present invention will be described in detail with reference to the accompanying drawings. In describing the present invention, well-known functions or constructions will not be described in detail when it is determined that they may obscure the gist of the present invention. Unless otherwise specified, all terms used in the present specification have the same meaning as the general meaning of the terms understood by a person with ordinary skill in the art to which the present invention belongs, and if the general meaning conflicts with the meaning of a term used in the present specification, the definition used in the present specification is applied.

A patient-specific breast implant 1 according to one embodiment of the present invention, when a breast-conserving surgery (BCS, also called lumpectomy or partial mastectomy) is performed to remove breast cancer while leaving as much normal breast tissue as possible, prevents deformation of the breast due to the depression of breast skin or adhesions of remaining breast tissues after the BCS, shortens surgery time, and facilitates securing breast symmetry, thereby ultimately dramatically increasing the success rate of surgery and maximizing patient satisfaction.

FIG. 2 is a view illustrating a patient-specific breast implant 1 according to one embodiment of the present invention. Referring to FIG. 2, the patient-specific breast implant 1 includes a shell portion 10.

Among components of the patient-specific breast implant 1, the shell portion 10 is a component forming an outer wall. Preferably, the shell portion 10 has a shape complementary to the shape of an inner space S expanded by a breast tissue expander. Therefore, it is possible to shorten the breast reconstruction surgery time, dramatically increase the surgical success rate of the breast reconstruction surgery and avoid sacrificing additional body tissues.

The shell portion 10 includes a shape memory polymer. Since the shell portion 10 includes a shape memory polymer, when the patient-specific breast implant 1 having a shape complementary to the shape of a breast tissue removal site after lumpectomy or partial mastectomy is inserted into the body, the shape of the implant 1 can be permanently maintained while the body temperature changes within a body temperature changeable range. This facilitates breast symmetry and enables cosmetically satisfactory breast reconstruction.

The shape memory polymer includes a polymer material that is changeable in shape according to temperature change. This polymer material may be any one polymer that is certified to be harmless when inserted into the human body and is selected from among polynorbornene, polyisoprene, styrene-butadiene copolymer, polyurethane, polyethylene, and polyester-based polymers. For this reason, when the patient-specific breast implant 1 is implanted into the body, the shape of the implant 1 can be permanently maintained when the body temperature of the patient is within the normal body temperature range. Therefore, the patient's breast having a similar shape to the original breast can be reconstructed due to the shell portion 10 having a shape complementary to the shape of an area from which the patient's breast is removed.

Preferably, the polymer material may be a polyester-based polymer, and the polyester-based polymer may be any one or a combination of two or more that are selected from polyphosphazene, polyanhydride, poly acetal, polyorthoester, polyphosphoester, polyglycolide, poly-ε-caprolactone, polylactide, polycarbonate, and polyamide and which are certified to be harmless when inserted into the human body.

Referring to FIG. 2, the shell portion 10 includes a silicone film 11 and a shape memory film 13.

The silicone film 11 is a film made of silicone. Preferably, the silicone film 11 may be disposed inside the shape memory film 13 to be described later. A filler F may be disposed inside the silicone film 11. The filler F may be saline. Alternatively, the filler F may be silicone gel or a shape memory polymer certified to be harmless when inserted into the human body. When the filler F is the shape memory polymer, the material disposed outside the shell portion 10 is the same as that of the filler F.

The silicone film 11 may be formed by 3D printing. Since the silicone film 11 is formed by 3D printing, a silicone film having a shape complementary to the shape of the breast removed during the breast-conserving surgery (partial mastectomy) can be formed.

The shape memory film 13 is a film made of a shape memory polymer. Preferably, the shape memory film 13 may be formed through surface treatment on the outer surface of the silicone film 11. When the entire outer wall of the shell portion 10 is made of a shape memory polymer, the use of a relatively expensive shape memory polymer increases. In this case, the manufacturing cost of the patient-specific breast implant 1 may be dramatically increased. By forming the shape memory film 13 on the outer surface of the silicone film 11 through surface treatment of the silicone film 11, it is possible to reduce the manufacturing cost of the patient-specific breast implant 1. However, the silicone film 11 may not be included and only the shape memory film 13 made of a shape memory polymer may be included to facilitate securing breast symmetry and to enable cosmetically satisfactory breast reconstruction even when a narrow area of depression and skin contracture occur after breast-conserving surgery (i.e., partial mastectomy).

In addition, by forming the shape memory film 13 on the outer surface of the silicone film 11 through surface treatment of the silicone film 11, the shape memory film 13 is deformed according to the temperature so that the overall shape of the breast implant is changed to have a shape complementary to the shape of the inner space formed by a breast tissue expander.

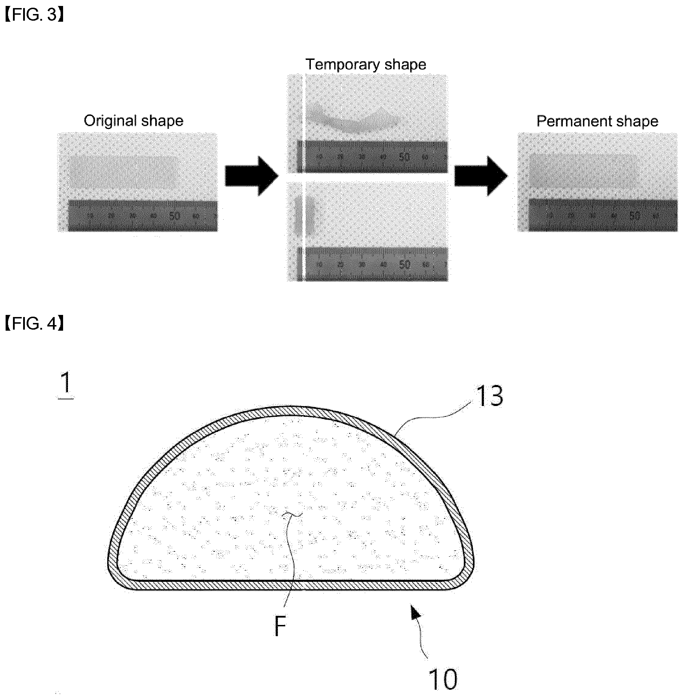

FIG. 3 is a view illustrating the original shape, a temporary shape, and a permanent shape of the shape memory polymer. Referring to FIG. 3, when it is assumed that the original shape of the shape memory polymer is rectangle as shown in FIG. 3, even though the shape memory polymer is crumpled or rolled to have a temporary shape shown in FIG. 3 due to an external force, the shape memory polymer returns to its original shape at a specific temperature. In the case of a shape memory polymer that returns to the original shape thereof within the body temperature range, the shape memory polymer can maintain the original shape thereof as a permanent shape within the body.

FIG. 4 is a view illustrating a patient-specific breast implant 1 having a shell portion made of only a shape memory film, according to another embodiment of the present invention. In this embodiment, the patient-specific breast implant 1 is formed such that the shell portion 10 is made of only a shape memory film 13.

That is, the shell portion 10 does not include the silicone film 11 and includes only the shape memory film 13 made of a shape memory polymer, in which the shape memory film 13 constitutes the outer wall of the breast implant 1.

In this case, the shell portion 10 may be directly formed through 3D printing using a 3D printing technology. Alternatively, a metal mold having a shape corresponding to a missing portion is first manufactured, and the shape memory film may be formed to have the same shape as the metal mold.

Therefore, when the patient-specific breast implant 1 is implanted in a chest region of a patient, the breast implant 1 changes into a shape memorized according to temperature so that the breast implant 1 can be precisely fitted into the inner space secured by a breast tissue expander.

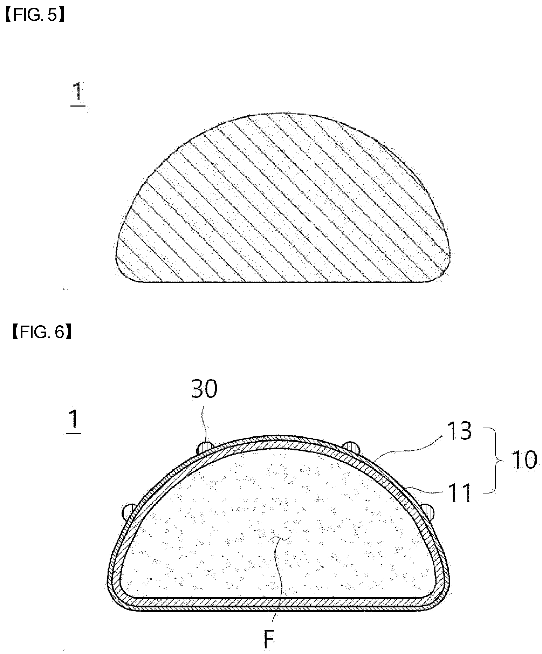

FIG. 5 is a view illustrating a patient-specific breast implant composed only of a shape memory polymer without including a silicone film or filler, according to a further embodiment of the present invention. Referring to FIG. 5, the entirety of the patient-specific breast implant 1 is formed of only a shape memory polymer through 3D printing rather than the patient-specific breast implant 1 is formed in a manner that a film is formed first and then the inside of the film is filled with filler. That is, it is possible to construct the patient-specific breast implant 1 in which both the shell portion 10 and the filler F are formed only of a shape memory polymer, and a 3D printing method may be used as a method of manufacturing the same.

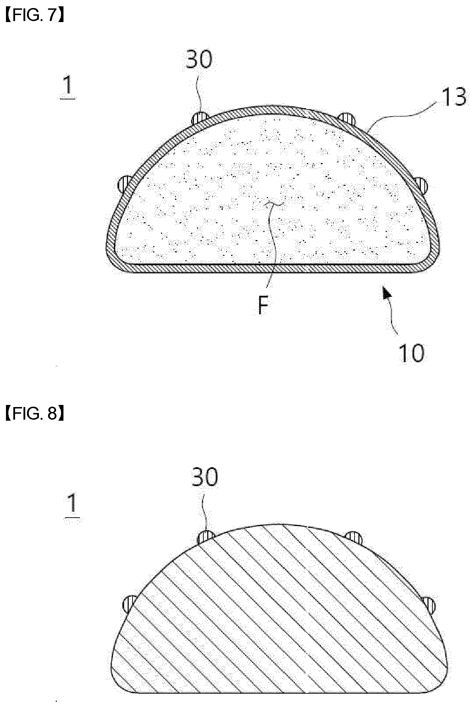

FIGS. 6 and 7 are views illustrating a patient-specific breast implant according to a further embodiment of the present invention. According to the present embodiment, the patient-specific breast implant 1 includes a fixing portion 30 formed on the shell portion 10. In the case of FIG. 6, the fixing portion 30 is provided on the patient-specific breast implant 1 of FIG. 2. In the case of FIG. 7, the fixing portion 30 is provided on the patient-specific breast implant 1 of FIG. 5. In order to avoid redundant description, only the newly added fixing portion 30 will be described below.

The fixing portion 30 protrudes from the surface of the shell portion 10. The fixing portion 30 protruding from the surface of the shell portion 10 serves to fix the breast implant inserted in the inner space secured by a breast tissue expander so that the breast implant may not be rotated or unintentionally moved in the body.

Referring to FIGS. 6 and 7, the fixing portion 30 has a hemispherical shape. However, the shape of the fixing portion 30 is not particularly limited. The fixing portion 30 may have any shape if it can have the function described above.

In order to improve the fixing effect of the fixing portion 30, preferably, a plurality of fixing portions 30 may be provided on the shell portion 10.

FIG. 8 shows a case where the patient-specific breast implant 1 includes neither a silicone film nor filler but is composed of only a shape memory polymer, and the fixing portion 30 is provided on this patient-specific breast implant 1. The patient-specific breast implant 1 may be configured as illustrated in FIG. 8. Alternatively, the fixing portion 30 as well as the patient-specific breast implant 1 may be made of a shape memory polymer. In this case, the patient-specific breast implant 1 and the fixing portion 30 are both formed through 3D printing.

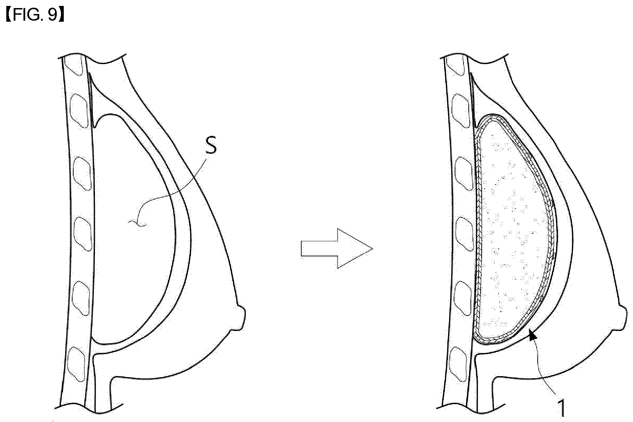

FIG. 9 is a view illustrating a state in which the present invention is used. Referring to FIG. 9, when a breast-conserving surgery (i.e., partial mastectomy) that minimizes the removal of the surrounding healthy tissue is performed, a small breast tissue expander is inserted into the area where the breast has been removed, and the inner space S is enlarged by expanding only the tissue in the desired area.

Thereafter, the patient-specific breast implant 1 having a shape complementary to the shape of the inner space S created by the small breast tissue expander is inserted into the inner space S. The breast implant 1 may completely fill the inner space S. As a result, the contour of the entire breast is not distorted, and a breast with a shape similar to the original breast shape can be reconstructed cosmetically.

## Claims

1. A patient-specific breast implant (1) comprising a shell portion (10) constituting an outer wall, the shell portion (10) having a shape complementary to a shape of an inner space (901, S) secured by a breast tissue expander (90),
wherein the shell portion (10) comprises a shape memory polymer;
wherein the shape memory polymer comprises a polymer material that is changeable in shape according to temperature change.

2. The patient-specific breast implant (1) of claim 1, wherein the polymer material is any one that is selected from poly-norbornene, polyisoprene, styrene-butadiene copolymer, polyurethane, polyethylene, and polyester-based polymer and which is certified to be harmless when inserted into the human body.

3. The patient-specific breast implant (1) of claim 2, wherein the polymer material is the polyester-based polymer, and the polyester-based polymer is any one or a combination of two or more that are selected from polyphosphazene, polyanhydride, poly acetal, polyorthoester, polyphosphoester, polyglycolide, poly-ε-caprolactone, polylactide, polycarbonate, and polyamide and which are certified to be harmless when inserted into the human body.

4. The patient-specific breast implant (1) of claim 1, wherein the shell portion (10) comprises a silicone film (11) made of silicone and a shape memory film (13) made of a shape memory polymer.

5. The patient-specific breast implant (1) of claim 4, wherein the shape memory film (13) is formed on an outer surface of the silicone film (11) through surface treatment.

6. The patient-specific breast implant (1) of claim 5, wherein the silicone film (11) is formed through 3D printing.

7. The patient-specific breast implant (1) of claim 1, wherein the shell portion (10) is made of only a shape memory film (13) and is formed through 3D printing.

8. The patient-specific breast implant (1) of claim 1, wherein a filler (F) and the shell portion (10) are made of only a shape memory polymer.

9. The patient-specific breast implant (1) of claim 8, wherein the filler (F) and the shell portion (10) are made of only a shape memory polymer and are formed through 3D printing.

10. The patient-specific breast implant (1) of any one of claims 1 to 9, further comprising a fixing portion (30) protruding from the surface of the shell portion (10).

## Patentansprüche

1. Patientenspezifisches Brustimplantat (1) mit einem eine Außenwand bildenden Mantelabschnitt (10), wobei der Mantelabschnitt (10) eine Form aufweist, die komplementär zu einer Form eines durch einen Brustgewebeexpander (90) gesicherten Innenraums (901, S) ist,
wobei der Mantelabschnitt (10) ein Formgedächtnispolymer aufweist;
wobei das Formgedächtnispolymer ein Polymermaterial aufweist, dessen Form in Abhängigkeit von Temperaturänderungen veränderbar ist.

2. Patientenspezifisches Brustimplantat (1) nach Anspruch 1, wobei das Polymermaterial ein beliebiges Material ist, das aus Polynorbornen, Polyisopren, Styrol-Butadien-Copolymer, Polyurethan, Polyethylen und Polyester-basiertem Polymer ausgewählt ist und das als unbedenklich beim Einbringen in den menschlichen Körper zertifiziert ist.

3. Patientenspezifisches Brustimplantat (1) nach Anspruch 2, wobei das Polymermaterial das Polyester-basierte Polymer ist und das Polyester-basierte Polymer eines oder eine Kombination von zwei oder mehr ist, die ausgewählt sind aus Polyphosphazen, Polyanhydrid, Polyacetal, Polyorthoester, Polyphosphoester, Polyglycolid, Poly-ε-caprolacton, Polylactid, Polycarbonat und Polyamid ausgewählt ist und das als unbedenklich beim Einbringen in den menschlichen Körper zertifiziert ist.

4. Patientenspezifisches Brustimplantat (1) nach Anspruch 1, wobei der Mantelabschnitt (10) einen Silikonfilm (11) aus Silikon und einen Formgedächtnisfilm (13) aus einem Formgedächtnispolymer aufweist.

5. Patientenspezifisches Brustimplantat (1) nach Anspruch 4, wobei der Formgedächtnisfilm (13) durch Oberflächenbehandlung auf einer Außenfläche des Silikonfilms (11) gebildet ist.

6. Patientenspezifisches Brustimplantat (1) nach Anspruch 5, wobei die Silikonfolie (11) durch 3D-Druck gebildet wird.

7. Patientenspezifisches Brustimplantat (1) nach Anspruch 1, wobei der Mantelabschnitt (10) nur aus einem Formgedächtnisfilm (13) besteht und durch 3D-Druck gebildet ist.

8. Patientenspezifisches Brustimplantat (1) nach Anspruch 1, wobei ein Füllstoff (F) und der Mantelabschnitt (10) nur aus einem Formgedächtnispolymer bestehen.

9. Patientenspezifisches Brustimplantat (1) nach Anspruch 8, wobei der Füllstoff (F) und der Mantelabschnitt (10) nur aus einem Formgedächtnispolymer bestehen und durch 3D-Druck hergestellt werden.

10. Patientenspezifisches Brustimplantat (1) nach einem der Ansprüche 1 bis 9, das ferner einen Befestigungsabschnitt (30) aufweist, der von der Oberfläche des Mantelabschnitts (10) herausragt.

## Revendications

1. Implant mammaire spécifique au patient (1) comprenant une partie enveloppe (10) constituant une paroi externe, la partie enveloppe (10) ayant une forme complémentaire à une forme d'un espace interne (901, S) fixé par un extenseur de tissu mammaire (90),
dans lequel la partie enveloppe (10) comprend un polymère à mémoire de forme ;
dans lequel le polymère à mémoire de forme comprend un matériau polymère dont la forme peut être modifiée selon un changement de température.

2. Implant mammaire spécifique au patient (1) selon la revendication 1, dans lequel le matériau polymère est un quelconque qui est choisi parmi le polynorbornène, le polyisoprène, le copolymère styrène-butadiène, le polyuréthane, le polyéthylène, et un polymère à base de polyester et qui est certifié inoffensif lorsqu'il est inséré dans le corps humain.

3. Implant mammaire spécifique au patient (1) selon la revendication 2, dans lequel le matériau polymère est le polymère à base de polyester, et le polymère à base de polyester est l'un quelconque ou une combinaison de deux ou plus qui sont choisis parmi le polyphosphazène, le polyanhydride, le polyacétal, le polyorthoester, le polyphosphoester, le polyglycolide, le poly-ε-caprolactone, le polylactide, le polycarbonate et le polyamide et qui sont certifiés inoffensifs lorsqu'ils sont insérés dans le corps humain.

4. Implant mammaire spécifique au patient (1) selon la revendication 1, dans lequel la partie enveloppe (10) comprend un film de silicone (11) constitué de silicone et un film à mémoire de forme (13) constitué d'un polymère à mémoire de forme.

5. Implant mammaire spécifique au patient (1) selon la revendication 4, dans lequel le film à mémoire de forme (13) est formé sur une surface externe du film de silicone (11) par traitement de surface.

6. Implant mammaire spécifique au patient (1) selon la revendication 5, dans lequel le film de silicone (11) est formé par impression 3D.

7. Implant mammaire spécifique au patient (1) selon la revendication 1, dans lequel la partie enveloppe (10) est constituée uniquement d'un film à mémoire de forme (13) et est formée par impression 3D.

8. Implant mammaire spécifique au patient (1) selon la revendication 1, dans lequel une substance de remplissage (F) et la partie enveloppe (10) sont constituées uniquement d'un polymère à mémoire de forme.

9. Implant mammaire spécifique au patient (1) selon la revendication 8, dans lequel la substance de remplissage (F) et la partie enveloppe (10) sont constituées uniquement d'un polymère à mémoire de forme et sont formées par impression 3D.

10. Implant mammaire spécifique au patient (1) selon l'une quelconque des revendications 1 à 9, comprenant en outre une partie de fixation (30) faisant saillie à partir de la surface de la partie enveloppe (10).
